# EUROPEAN PATENT APPLICATION

(11) **EP 1 157 667 A2**
(43) Date of publication of application: **28.11.2001**
(21) Application number: 01304618.0
(22) Date of filing: 24.05.2001
(51) Int. Cl.: A61B 18/12

(54) **Electrosurgical generator with RF leakage reduction**

(30) Priority: 25.05.2000 US 578957
(71) Applicant: ETHICON ENDO-SURGERY, INC., Cincinnati, OH 45242-2839 (US)
(72) Inventor: Bowers, William J., Highland Ranch, CO 80126 (US)
(74) Representative: Mercer, Christopher Paul

(57) **Abstract**

An electrosurgical generator having circuitry and a method for reducing RF leakage to an acceptable level. The invention utilizes an isolated output configuration and two separate methods of monitoring the output waveforms. The first method utilizes the output voltage to determine if the generator should operate in a RF leakage reduction mode or not. If the output voltage applied to the patient is low, lower than a preset level, then the generator will operate normally. If the output voltage applied to the patient is higher than the preset level, then the generator will operate in the leakage reduction mode. The second method utilizes the output current to determine whether the generator should operate in the RF leakage reduction mode. If the output current applied to the patient is low, lower than a preset level, the generator will operate in the RF leakage reduction mode. If the output current applied to the patient is higher than the preset level, then the generator will operate normally.

## Description

### BACKGROUND OF THE INVENTION

### 1. Field of the Invention

The present invention relates to electrosurgical generators, and in particular to an electrosurgical generator with RF leakage reduction.

### 2. Background Information

The use of RF energy to cut and coagulate tissue during a surgical procedure is well known. Anytime an electrosurgical generator is activated, varying amounts of RF leakage are produced. Power setting, RMS (root mean squared) voltages, accessories and physical layout of the system cause the amount of RF leakage to vary. All of these variables, when set in the worse case condition, can cause a high level of RF energy that could cause a patient and/or operating room personnel to receive an RF bum. (A RF bum is caused by a high current density flowing through a small amount of tissue area for a relative long period of time in an area that is not intended to have a clinical affect applied to it). These RF bums can vary from slight reddening to severe bums requiring surgical intervention..

To address this problem, test agencies have adopted specific tests to accurately measure the amount of RF leakage produced by an electrosurgical generator. An example of a test agency that has adopted the RF leakage test is International Electrotechnical Commission (IEC). IEC 60601-2-2 has defined a test setup and the maximum amount of RF leakage allowed for this test setup. Under the prescribed test, the maximum amount of RF leakage is 150 milliamps.

### SUMMARY OF THE INVENTION

The electrosurgical generator of the present invention utilizes an isolated output configuration designed to reduce RF leakage. In addition, the invention comprises a method for sensing output parameters to reduce RF leakage. Two separate methods of monitoring the output waveforms are disclosed. The first method utilizes the output voltage to determine if the generator should operate in a RF leakage reduction mode or not. If the output voltage applied to the patient is low, lower than a preset level, then the generator will operate normally. If the output voltage applied to the patient is higher than the preset level, then the generator will operate in the leakage reduction mode.

The second method utilizes the output current to determine whether the generator should operate in the RF leakage reduction mode. If the output current applied to the patient is low, lower than a preset level, the generator will operate in the leakage reduction mode. If the output current applied to the patient is higher than the preset level, then the generator will operate normally.

### BRIEF DESCRIPTION OF THE DRAWINGS

Fig. 1 is a schematic diagram illustrating the general functions of an electrosurgical generator;
Fig. 2 is a load curve graphing output power to output load impedance of the electrosurgical generator;
Fig. 3 illustrates typical RF logic waveforms;
Fig. 4a illustrates the sensed peak output voltages of the preset levels for the CUT mode at various power settings;
Fig. 4b illustrates the sensed peak output voltages of the preset levels for the COAG mode at various power settings;
Fig. 5 is a schematic diagram similar to Fig. 1 adding voltage sensing and peak detecting;
Fig. 6 illustrates the RF drive timing;
Fig. 7 illustrates an input and output of a peak detector when operating the generator into an open circuit;
Fig. 8 illustrates a typical input/output of the peak detector when operating the generator into a loaded condition;
Fig. 9 illustrates a normal operating condition relating to the peak detector while delivering power to the load;
Fig. 10 is a schematic diagram similar to Fig. 1 adding current sensing and average current detecting;
Fig. 11 is a schematic diagram of a voltage peak detector circuit;
Fig. 11a is a schematic diagram showing the connection of the voltage peak detector to the output bandpass filter;
Fig. 12 is a schematic diagram of an average current detector circuit;
Fig. 12a is a schematic showing the connection of the average current sense circuit connected to the output of the generator;and
Fig. 13 illustrates an output of the average current detector circuit.

### DESCRIPTION OF THE PREFERRED EMBODIMENT

Before explaining the present invention in detail, it should be noted that the invention is not limited in its application or use to the details of construction and arrangement of parts illustrated in the accompanying drawings and description, because the illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

### I. General Electrosurgical Operation

### System Operation

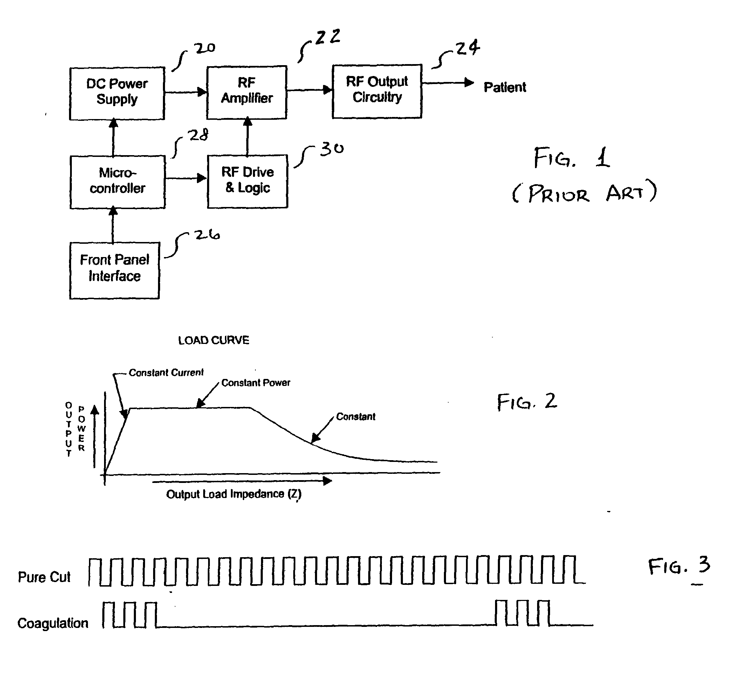

In order to understand the operation of the invention a general description of the power control system operation of an electrosurgical generator is required. Fig. 1 shows the main functions that describe the operation of the RF Leakage Reduction Algorithm. Additional block diagram functions are required to explain the complete operation of an electrosurgical generator but are not included in this disclosure, but are well known to those skilled in the art.

### DC Power Supply

The DC power supply 20 converts AC line voltage to a DC voltage that drives the RF amplifier 22. The power supply 20 regulates the output current, power and voltage. (Prior art shows that the same regulation properties can be achieved by a Pulse Width Modulation (PWM) technique on a RF amplifier. This disclosure does not use this PWM technique, but the algorithm described within will operate using the PWM technique). As the RF energy is delivered, the power supply 20 determines the appropriate regulation mode (current, power or voltage) in which it should operate. The graph of Fig. 2 provides a better understanding of the regulation modes.

Referring to Fig. 2, constant current occurs at the lower impedances when the output requires higher current than a pre-set value. The different modes of operation, such as cut or coagulation, require different constant current limits. As long as the output impedance is low the DC power supply 20 will regulate to the constant current. When the output impedance increases, the DC power supply 20 will change to a constant power regulation. Constant power operates within the medium range of impedances and limits the output power for each power setting. This constant power allows the same amount of power to be delivered to the patient as long as the impedance is within the proper range. As the output impedance continues to increase, the DC power supply 20 begins to regulate with a constant voltage. The constant voltage limits the output DC voltage that is delivered to the RF amplifier 22. In turn, the output power reduces in an inverse relationship (¹/Z) to the output impedance. At the point in which the system changes from one regulation mode to the next, a "roll-off" point can be defined. For example, if the Cut mode transitions from the power regulation mode to the voltage regulation mode at 800 ohms, the cut mode is said to have an upper "roll-off' point of 800 ohms.

### RF Amplifier

The RF amplifier 22 converts the DC voltage to a high frequency, energy. Any type of RF amplifier can be used to create the electrosurgical energy, i.e., Full Bridge, Single Ended, Push-Pull, etc. By controlling the RF energy, as described in the DC power supply 20 section and by controlling the output waveform, different clinical effects are produced. The cut mode is produced by creating a continuous, approximately 500 kHz RF waveform. Applying a duty cycle to the waveforms produces Blend (Cutting with Hemostasis), Coagulation (Fulguration and Contact) and Bipolar Coagulation modes. Different clinical effects can be achieved depending on the duty cycle and the "roll-off" points of the mode of operation.

### RF Output Circuitry

The RF output circuitry 24 begins with the output transformer and ends at the operating site. All RF amplifiers have an output transformer to convert the low primary voltage to a higher output voltage. The cut modes require less voltage, but higher power levels than the coagulation modes. The output transformer steps the primary voltage to a point where the requirements of the roll-off points at maximum power settings are met. In the coagulation mode, especially in the Spray or Fulguration mode, the turns ratio is much higher than in the cut modes. This allows for a very high voltage level when the generator is operated into a high impedance in the voltage regulation mode. The high voltage is required for creating and maintaining arcs to the operating site.

In most cases, the RF output circuitry 24 requires a high frequency filter. (Many Single Ended type amplifiers operate into a "tune tank", thus producing sinusoidal waveforms naturally.) The filter coverts the input waveform, basically a square wave, into a sinusoidal waveform. By converting the output to a sinusoidal waveform, the problem with RF leakage is reduced. In most cases, however, RF leakage is still higher than the maximum allowed RF leakage current a specified in IEC 60601-2-2.

In addition, the RF output circuitry 24 requires relays to direct the RF energy to the appropriate output accessory and, in turn, to the operative site.

### Front Panel Interface

The Front Panel Interface 26 is simply the interface between the operating room personnel and the generator. The operating room personnel instruct the generator of the appropriate mode and power setting for the clinical procedure. In tum, the front panel displays the requested information and provides activation information to the operating room personnel. In most generators, when the operating room personnel activate a mode, the front panel initiates an indicator to inform the user which mode is operated.

### Microcontroller

The microcontroller 28 is the heart of the system. (In some simple electrosurgical generators, a microcontroller is not used. Instead, logic systems are used to determine the way the system is operating). Its main function is to determine the system operation depending on the input received. In addition, the microcontroller 28 monitors the safety features of the system. If a safety system shows a problem, the microcontroller 28 determines and implements the appropriate action.

The microcontroller 28 determines the level of DC voltage required to produce a given mode of operation, RF drive waveforms, "roll-off" points to determine the load curve, and many other functions of the generator. By using a microcontroller 28, many functions, input and output, can be conducted simultaneously. For example, when the generator is activated in the Spray Coag mode, an input to the microcontroller 28 can monitor the peak voltage of the output waveform. If the output voltage reaches a pre-determined level the RF drive signal could be modified. By adjusting the RF drive waveform, different physiological effects can be obtained.

### RF Drive & Logic

Electrosurgical generators develop the RF drive signals many different ways. Some use discrete transistors, some use logic systems, while others use a combination of microcontroller and logic to determine the RF drive pulses. In all electrosurgical generators different waveforms are generated for given modes of operations by the RF drive and logic circuit 30. For example, a pure cut waveform would have continuous train of pulses delivered to the RF amplifier 22. A coagulation waveform would have a short duration of pulses (in some cases only one pulse) and a long duration of off time, which provides high peak energy while producing low RMS power to the surgical site required for coagulation. The train of pulses will operate at the operating frequency of the RF amplifier 22. All electrosurgical generators operate between 300kHz and 5MHz, with the majority operating around 500kHz. Typical RF logic waveforms are shown in Fig. 3.

The RF drive receives pulses from the RF logic and increases the energy required to operate the RF amplifier 22. For example, the power MOSFETs used in the RF amplifier 22 requires a switching signal that is positive 15 volts to properly tum the device on and off. The RF drive and logic circuit 30 produces these required voltage levels. Many different circuits are well known to properly operate the power MOSFET transistors inside the RF amplifier.

### II. RF Leakage Reduction Algorithm

### Voltage Sensing Method

### Output Voltage Characteristics

Output voltage varies depending upon the output load characteristics under which the generator is operating. If the output load impedance is low enough to operate in the constant current or the constant power portion of the output load curve, the voltage will remain relatively low. While the generator operates in these portions of the load curve the power supply 20 will adjust the DC voltage, which in turn, will adjust the output voltage.

When the load impedance reaches values high enough to operate in the constant voltage portion of the load curve, the DC power locks into a constant voltage mode. At this point the output power begins to roll off as shown Fig. 2. Different modes of operations require different roll off points. For example, the roll off point for the Cut mode could be around 800 ohms while the Blend mode rolls off at 1500 ohms. The desired clinical effect and the output characteristics of the mode determine each roll off point.

As the impedance increases beyond the roll off point, the DC power supply 20 remains at the constant voltage but the peak voltage of the output waveform continues to increase. The increased "Q" of the output filter and the overshoot characteristics of the pulsed output waveform causes an increase in peak voltage. The Cut waveform increases only because of the increased "Q" of the output filter since the Cut mode is not pulsed. Since the higher impedances, greater than 2000, provide a higher "Q" for the output filters the increase in peak voltage is significant. This large open circuit peak voltage is a strong indicator that the generator is operating in or close to an open circuit load. This is the worst-case condition and carries with it an increased risk of a RF bum.

Shown in Fig. 4a and 4b are two graphs that show the sensed output voltages for the Cut mode and the Spray Coag mode, respectively, in the present generator. Each graph shows the output voltage of the peak detector (explained below) for the 2000 ohms load, lower curve, and the open circuit load, upper curve. It should be noted that the Spray mode exhibits a much higher differential between the two loads than the Cut mode. This is because the Cut mode is not a pulsed drive signal and the waveform will increase only as a function of the increased "Q" of the output filter.

In addition to the actual measured voltages in the Cut mode, the Cut RF Transfer Function shows the derived mathematical function as determined by a curve-fitting program. These mathematical functions are used to tell the microcontroller whether or not to operate in the RF leakage reduction mode. Although not mandatory, the use of mathematical models does make implementation into the software much easier. In the case of the Spray mode, "switching steps" can be used to tell the microcontroller in which mode to operate.

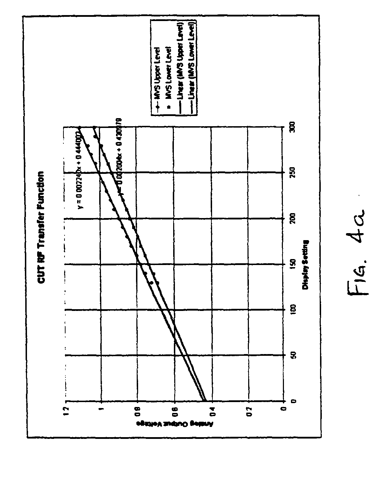

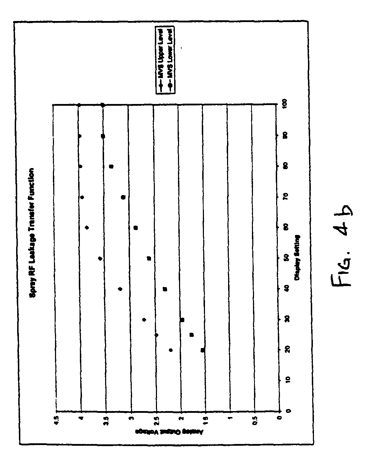

Fig. 5 illustrates the RF leakage topology. The voltage sense 32 and voltage peak detector 34 circuit blocks were added to the original block diagram of Fig. 1.

To sense the output voltage of the generator, a high frequency, high voltage sensing method must be employed. Several different methods are available but the present generator utilizes a high frequency, high voltage sensing transformer 35 to sense the output voltage. The "turns ratio" used in the sensing transformer is 1:48 for the present generator. This value can easily be adjusted to adapt to different sensing configurations.

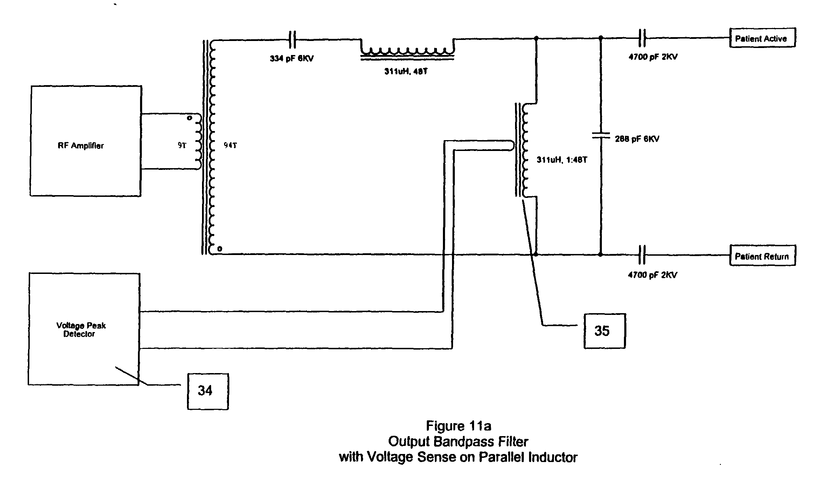

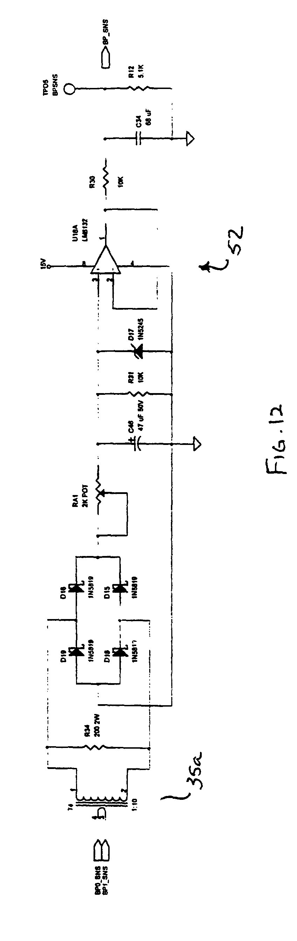

The output voltage sense transformer 35 is applied to a voltage peak detector 34, shown in Fig. 11 and Fig. 11a. The sensing transformer also acts as the parallel leg of the output filter as shown in Fig. 11a. Peak detectors are used to accurately sense and hold the measured peak voltage of the output waveform. As the output voltage pulses are applied to the peak detector, a DC voltage that represents the highest peak is developed on the output of the detector. In this application the actual input/output voltage ratio is approximately 1 to 1000. Therefore, a 1-volt DC signal on the output of the peak detector represents a 1000-volt peak on the output of the generator.

### Application of the Output of the Peak Detector into the Microcontroller

The output voltage of the peak detector is applied to an analog input of the microcontroller 28. Alternatively, it should be noted that the output of the peak detector could be applied to an "off-the-shelf" A to D converter. In turn, the output of the A to D converter is then applied to the digital bus. The microcontroller 28 converts the analog voltage to a digital number that represents the analog voltage. This digital number is then compared to a reference level set up in the software at each power setting. If the voltage is greater than the upper level then the microcontroller 28 will operate in the RF leakage reduction mode. If the voltage is lower than the lower level then the microcontroller 28 will operate in the normal mode. By using two reference levels, a hysteresis switching can be established. This will reduce the tendency for the generator to oscillate between the RF leakage reduction mode and the normal mode of operation. In some applications this hysteresis may not be required.

For each mode of operation, different reference tables are used to optimize switching between the RF leakage reduction mode and the normal mode of operation. Therefore, each power setting will have the appropriate reference level to operate properly.

### Application of the RF Leakage Reduction Mode on the RF Amplifier Circuitry

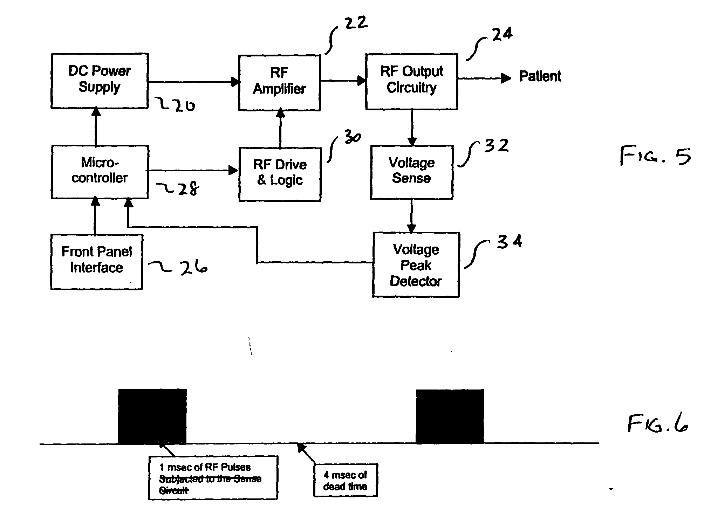

When the generator is activated into an open circuit, the peak voltage of the output waveform increases to a point above the upper reference voltage. For example, if the generator is operated in the Spray Coag mode at a power setting of 50 watts, the output of the peak detector would read approximately 3.6 volts (see the Spray Coag RF Leakage Transfer Function in Fig. 4b). If the upper reference voltage for this power setting is lower than the 3.6 volts, then the microcontroller 28 will switch the drive for the RF amplifier 22 into the RF leakage reduction mode. In this mode, the RF Drive and Logic 30 is cycled on and off. An exemplary cycle sequence is to turn the RF drive "on" for 1 millisecond and then "off" for 4 milliseconds. The one to four ratio is currently being used in the present generator but other values can be chosen for different applications such as 1 millisecond "on" and 5 milliseconds "off". The 1 millisecond "on" time gives the generator ample time to operate and relay the output conditions back the microcontroller 28. The 4 milliseconds "off" time was chosen to ensure that RF leakage is reduced to an acceptable level. A drawing of the RF output waveform driving the RF Amplifier is shown in Fig. 6, wherein the square wave is representative of a train of RF pulses.

With the drive waveform operating with 1 millisec "on" and 4 millisecs "off", a 20% duty cycle is generated. This 20% duty cycle is, in turn, applied to the output waveform. As a result, the RMS output waveform is reduced to approximately 20% of the original 100% on time waveform. Since RF leakage is a function of the RMS voltage applied to the operating site, an output waveform that has a reduction of the RMS voltage to 20% of the original RMS voltage, a much lower value of RF leakage is achieved. As would be apparent to those skilled in the art, the value of RF leakage might not be 20% of the original RF leakage because of the many variables that effect RF leakage, but it will be significantly reduced.

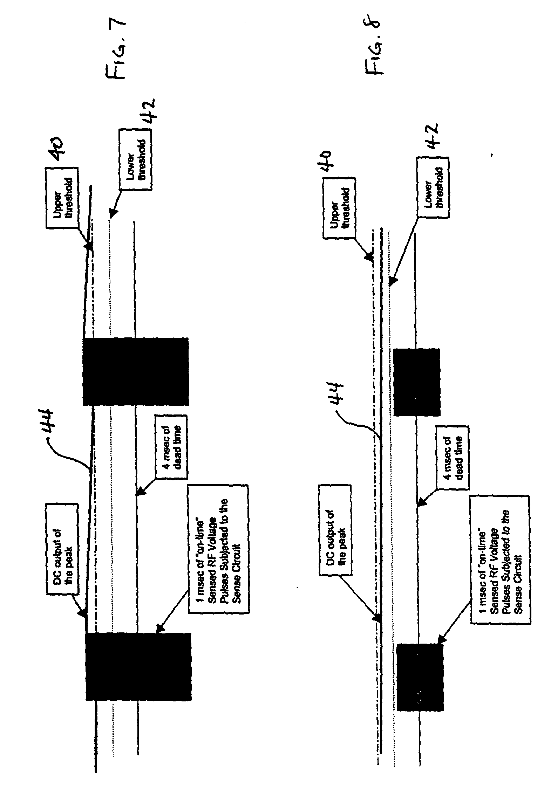

Referring to Fig. 7, as long as the generator remains in an open circuit condition, the peak voltage will remain high enough to keep the DC voltage 44 from the voltage peak detector 34 higher than the upper threshold 40. The microcontroller 28 will thus keep the generator operating in the RF leakage reduction mode. As soon as the output is operated into a loaded condition (the active electrode is cutting or coagulating tissue), the peak voltage will drop below the lower threshold 42 and the microcontroller 28 will begin to operate the generator in the normal mode.

Continuing the example of the 50-watt setting of the Spray mode, the peak voltage represented by the voltage peak detector 34 will read approximately 2.6 volts when the output is delivered into 2000 ohms. This difference between the open circuit voltage (3.6 volts) and the loaded voltage (2.6 volts) allows for a differential voltage of approximately 1.0 volts. In order for the microcontroller 28 to operate properly, thresholds must fall between these two levels. At the 50-watt setting in the Spray mode, if the upper level is set to 3.0 volts and the lower level is set to 2.9 volts the microcontroller 28 will properly switch in and out of the RF leakage reduction mode. Each power setting and mode of operation will require a new set of thresholds to operate properly.

### Input / Output Signals of the Peak Detector

If the voltage peak detector 34 is subjected to an open circuit voltage (while at a high power setting), the DC voltage presented to the microcontroller 28 will be higher than a preset voltage. (This voltage may either be one or several fixed voltages or a formula to represent the threshold voltages.) When the DC voltage exceeds the preset voltage the microcontroller 28 will begin to deliver bursts of drive pulses instead of continuous drive pulses. These bursts of RF pulses on the output will produce a lower RMS voltage while keeping the peak voltage high. The one millisecond "on time" will allow the generator to produce an arc when the electrode is close enough to the tissue. In order to reduce the RF leakage to an acceptable level, the RF drive should be turned off for approximately 4 millisecs. This will produce a 20% duty cycle. The diagram shown in Fig. 7 shows the input and the output of the-voltage peak detector 34 while the generator is operating in an open circuit.

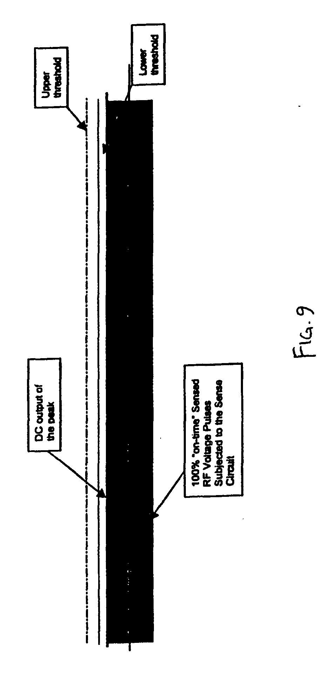

While the generator is operating in the RF leakage reduction mode, the voltage peak detector 34 continuously monitors the output waveform. If the output operates in a loaded condition, the peak voltage will drop. The drop in output voltage is applied to the voltage peak detector 34 and begins to drop the peak detector's output. The amount of time it takes for the output voltage to decrease below the lower threshold is defined by the RC time constant of the output circuitry of the peak detector. The design of the RC time constant will determine the desired clinical effect. If the time constant is too short, the generator may continuously switch between the RF leakage reduction mode and the normal mode of operation or it may switch under a noise pulse. If the time constant is too long a "buzzing" noise may be heard at the operating site. This will be an annoyance to the operator. Until the voltage drops below the lower threshold 42, the diagram of Fig. 8 shows the typical input/output characteristics of the peak detector 34. Since the output peak voltage is below the output voltage of the peak detector 34, the peak detector 34 will not "pump-up" the peak detector's output voltage. The microcontroller 28 will continue to operate the system in the RF leakage reduction mode.

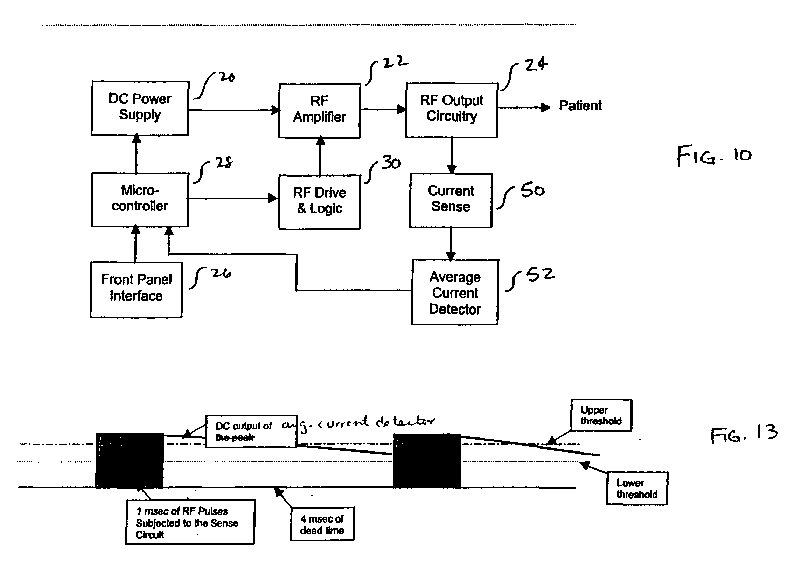

Eventually the peak detector's output voltage will reduce to the point that is below the lower threshold 42. At that time, the microcontroller 28 will switch the output characteristics to the normal operating mode of operation, Fig. 9. As long as the output is operated into low impedances, the generator will remain in this mode of operation and the output voltage of the peak detector 34 will remain below the upper threshold. As soon as the generator is operated into an open circuit, the RF leakage reduction mode will begin to operate once again.

### Current Sensing Method

By using current as the sensing variable, the same characteristics for RF leakage reduction can be achieved. The current sensing method uses either peak detection or an RMS/Averaging sensing method. For this application it was simpler to apply the averaging sensing system.

Depending on the output characteristics into which the generator is operating, the average output current will vary. If the output load impedance is low enough to operate in the constant current or the constant power portion of the output load curve the current will remain relatively high. While the generator operates in these portions of the load curve the power supply 20 will adjust the DC voltage, which in turn, will adjust the output current.

When the load impedance reaches values high enough to operate in the constant voltage portion of the load curve, the average output current will be low. As the load impedance increases to higher impedances, the current continues to reduce. At an open circuit condition the current reduces close to zero current (the only current that is produced is a result of the leakage current).

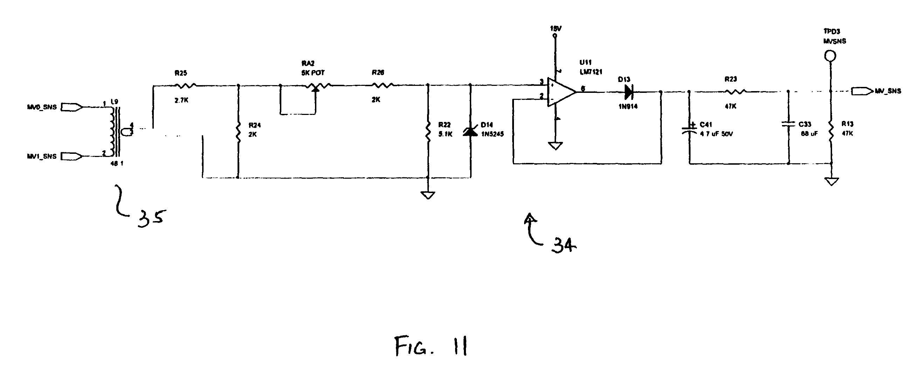

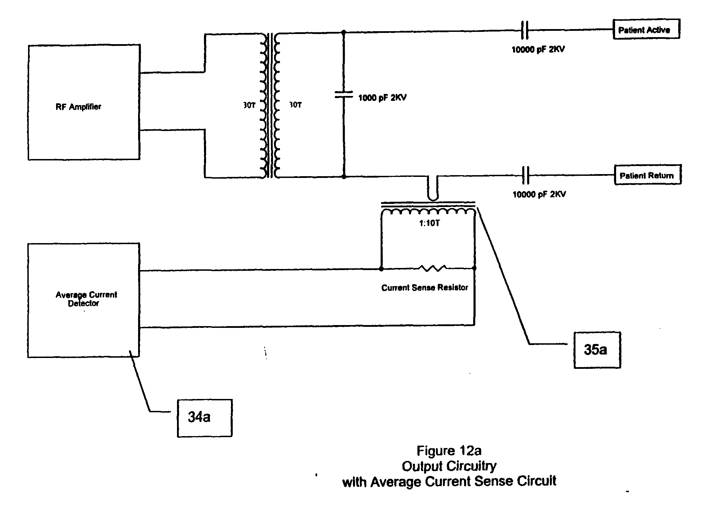

The block diagram shown in Fig. 10 shows the major circuit functions to make up the current sensing method. Note that the configuration of the blocks is very similar to the voltage sensing method. The schematic for the average current detector 52 is shown in Fig. 12.

### Application of the RF Leakage Reduction Mode on the RF Amplifier Circuitry

The algorithm for RF leakage reduction is the same as in the voltage mode, except current is measured and compared against thresholds. Tables contained within the microcontroller 28 are adjusted for the current sensing system. The rest of the algorithm remains the same.

Examples of the RF reduction algorithms follow below. The first describes the system that is currently used in the present generator for the monopolar mode. The monopolar mode uses the voltage-sensing algorithm. The second describes the system that is currently used in the present generator for the bipolar mode. The bipolar mode uses the current-sensing algorithm.

### RF Leakage Reduction Algorithm (Monopolar Only)

Referring to Fig. 13, the following is a description of the process how the RF Leakage Reduction circuitry and software functions in the generator for monopolar operation.

### Basic Operation

RF leakage occurs anytime an electrosurgical generator is operated. The amount of RF leakage produced by a generator is a function of many factors. IEC 60601-2-2 has defined a test setup and the maximum amount of RF leakage allowed for this test setup. The generator is an isolated output configuration and is designed to reduce the RF leakage as much as possible, but because of the high open circuit voltage the RF leakage is still higher than the IEC limit. This algorithm describes the method in which the RF leakage reduction will operate.

The way the generator operates, the highest level of RF leakage occurs in the open circuit mode. This is when the peak voltage and the RMS voltage of the output waveform are the highest. In order to reduce the RF leakage, the RMS voltage of the waveform must be reduced to a level that will pass the RF leakage specification. One method to reduce RF leakage is to lower the DC power supply 20 causing the RMS voltage to reduce. The peak voltage, however, will also reduce and in turn, the arc length, especially in the Spray mode, will also reduce. This is not the best way to solve the RF leakage problem. The disclosed RF leakage reduction algorithm will reduce the RMS of the waveform while maintaining the same peak voltage. By keeping the peak voltages high, the arc length will remain the same in the Spray mode.

A sample of the output voltage is applied to a voltage peak detector 34. In each mode, the open circuit voltage is significantly higher than the loaded voltage. When the generator is keyed, the output waveform is sampled and sent to the peak detection circuitry. This circuit converts the peak voltage to a DC level. The output capacitor of the peak detector charges quickly but discharges slowly. The rate of discharge is simply adjusted by the output R and C values. In general, see Fig. 13.

### Input / Output Signals of the Peak Detector

If the peak detector is subjected to open circuit voltage (while at a high power setting), the DC voltage presented to the microcontroller 28 will be higher than a preset voltage. This voltage may either be one or several fixed voltages or a formula to represent the threshold voltages. When the DC voltage exceeds the preset voltage the microcontroller 28 will begin to deliver bursts of drive pulses instead of continuous drive pulses. These bursts of RF pulses on the output will produce a lower RMS voltage while keeping the peak voltage high. The one millisecond "on time" will allow the generator to produce an arc when the electrode is close enough to the tissue. In order to reduce the RF leakage to an acceptable level, the RF drive should be turned off for approximately 4 millisecs. This will produce a 20% duty cycle.

### Sequence

1. After the RF is turned on, read the voltage from the peak detector.
2. If the voltage is below the upper level for the mode of operation and the power setting, leave the RF drive on 100% duty cycle. If the voltage is above the upper level for the mode of operation, remain 100% but continue to monitor the voltage, for example, every 15 msecsonds. Make 4 more readings. If the voltage stays above the upper level for the mode of operation, then start to deliver the 20% duty cycle of the output waveform.
3. Each time the software loop comes back around read the voltage from the peak detector.
4. If the voltage is above the lower level (after switching to 20% duty cycle) for the mode of operation and the power setting, leave the RF drive on 20% duty cycle. If the voltage is below the lower level for the mode of operation, then start to deliver the 100% duty cycle of the output waveform. If the voltage remains below the lower level for the mode of operation, then stay in the 100% duty cycle of the output waveform. Continuously monitor the peak detector's output voltage (each time the software loop comes back around). If the voltage is above the upper level for the mode of operation go back to step 2 and modify as described.
5. Continuously monitor and adjust the output waveform to adapt to the output characteristics.
6. Each mode will have a separate upper and lower limit. The table below indicates the fixed values.

**Table 1**

| **CUT Mode RF Leakage Switching Table (Output voltage of the Peak Detector)** | | |
|---|---|---|
| Power Setting (Watts) | Upper Threshold Limit (V) | Lower Threshold Limit (V) |
| 0 - 139 | None* | None |
| 140 - 300 (each power setting) | Formula (see below) | 95% of Upper Limit |

Cut upper limit formula: Analog Output Voltage = (0.00185*(Power Setting) + 0.4344)

For all Tables, where "None" is indicated for a power setting range, the RF leakage is well below the maximum allowed as specified by IEC; therefore, there is no reason to enable the RF leakage mode of the this invention.

**Table 2**

| **BLEND 1 Mode RF Leakage Switching Table (Output voltage of the Peak Detector)** | | |
|---|---|---|
| Power Setting (Watts) | Upper Threshold Limit (V) | Lower Threshold Limit (V) |
| 0 - 129 | None | None |
| 130 - 250 (each power setting) | Formula (see below) | 93% of Upper Limit |

Blend 1 upper limit formula:Analog Output Voltage = (0.004*(Power Setting) + 0.75463)

**Table 3**

| **BLEND 2 Mode RF Leakage Switching Table (Output voltage of the Peak Detector)** | | |
|---|---|---|
| Power Setting (Watts) | Upper Threshold Limit (V) | Lower Threshold Limit (V) |
| 0-124 | None | None |
| 125 - 250 | Formula (see below) with maximum voltage limit | 97% of Upper Limit |

Blend 2 upper limit formula:Analog Output Voltage = (0.00819*(Power Setting) + 0.7486)
Analog Output Voltage Limit = 2.05 V

**Table 4**

| **SPRAY Mode RF Leakage Switching Table (Output voltage of the Peak Detector)** | | |
|---|---|---|
| Power Setting (Watts) | Upper Threshold Limit (V) | Lower Threshold Limit (V) |
| 0 - 24 | None | None |
| 25 - 100 (each power setting) | Formula (see below) | - 0.3137 V of Upper Limit |

Spray upper limit formula: Analog Output Voltage = 0.00000256*(Power Setting)³-0.00087552*(Power Setting)² + 0.09844218*(Power Setting) + 0.03554674

**Table 5**

| **PIN POINT Mode RF Leakage Switching Table (Output voltage of the Peak Detector)** | | |
|---|---|---|
| Power Setting (Watts) | Upper Threshold Limit (V) | Lower Threshold Limit (V) |
| 0 - 99 | None | None |
| 100 - 120 (each power setting) | Formula (see below) with maximum voltage limit | 98% of Upper Limit |

Pin Point upper limit formula: Analog Output Voltage = (0.00846*(Display Setting) + 1.0072)

### RF Leakage Reduction Algorithm (Bipolar Only)

### Basic Operation

The Bipolar section operates differently than the monopolar section. Since the voltage peak detector 34 does not operate in the bipolar mode (the voltage sense wire is hooked into the monopolar section only), the current sense circuit of Fig. 12 determines whether or not to implement the RF leakage reduction algorithm in the bipolar mode. The basic operation of the RF leakage reduction will work the same as the monopolar mode but it will sense the output current instead of voltage. The output current is sensed by a current transformer 35a and delivered to the average current detector 52, Fig. 12a. This current is converted to a voltage that represents the output current and is delivered to the microcontroller for processing. This algorithm will require a hysteresis between the upper and lower switching limits. At given power settings current will be used to determine the upper and lower switching point in which the circuit will switch between the "normal" mode and the RF leakage mode. Each power setting will require a different switching point; therefore two formulas will be used to define the upper and lower switching points.

The two switch points will have different meanings depending on whether the system is in the normal mode or in the RF leakage reduction mode. If the generator is operating in the normal mode (current is relatively high) the algorithm uses the upper limit to switch into the RF leakage reduction mode. If the generator is operating in the RF leakage reduction mode then the algorithm uses the lower limit to switch out of the RF leakage reduction mode. Each time the generator switches into one or the other mode, the algorithm must stay in the switched mode until a new switching value is determined for the display setting. For example, the generator is set for 25 watts. Before the generator is keyed in the Bipolar mode, the algorithm determines the upper switching point. If current is below this upper level then the generator switches into the RF leakage reduction mode. The switching point is now the lower switching point for the given display setting. As soon as the current is above the lower limit for the 25 watt setting, then the generator switches back into the normal mode.

### Sequence

1. For each power setting determine the upper and lower "switching points" for that power setting.
2. After the RF is turned on, using the normal mode for the initial ON condition read the current from the current meter.
3. If the current is above the upper switching point for the display setting, leave the RF drive on the 100% duty cycle. Since the RF drive is in the normal mode then the upper limit is used to determine the switching point. If the current is below the upper switching point, remain at 100% but continue to monitor the current (you can come back to it every 10 msecs or so). Make 4 more readings. If the current stays below the switching point for the display setting, then start to deliver the 33% duty cycle of the output waveform.
4. Each time the software loop comes back around read the current from the current meter.
5. Since the generator is now operating in RF leakage reduction mode the generator must use the lower switch point to determine the point where the RF drive switches back into the normal mode. If the current is below the lower switching point (after switching to 33% duty cycle) for the power setting, leave the RF drive on 33% duty cycle. If the current is higher than the lower switching point, then start to deliver the 100% duty cycle of the output waveform. Once the RF drive switches back to the 100%, then the algorithm uses the upper limit once again. Continuously monitor the current meter's output voltage (each time the software loop comes back around). Go back to step 2 and modify as described.
6. Continuously monitor and adjust the output waveform to adapt to the output characteristics.
7. The bipolar mode will have an upper and lower limit. The table below indicates the formulas.

**Table 6**

| **RF Leakage Switching Table (Output voltage of the Peak Detector)** | | |
|---|---|---|
| Power Setting (Watts) | Upper Switching Point (I) | Lower Switching Point (I) |
| 0 - 5 | None | None |
| 5 - 50 (each power setting) | Upper Limit Formula (see below) | Lower Limit Formula (see below) |

Upper Limit Formula = 0.0000169*(Power Setting) ³ - 0.0018677*(Power Setting) ² +0.0926775*(Power Setting) +0.0483468
Lower Limit Formula = 0.0000077*(Power Setting) ³ - 0.0008886*(Power Setting) ² +0.0491257*(Power Setting) +0.0276647

It will be apparent from the foregoing that, while particular forms of the invention have been illustrated and described, various modifications can be made without departing from the spirit and scope of the invention. Accordingly, it is not intended that the invention be limited, except as by the appended claims.

## Claims

1. An electrosurgical generator for supplying radio frequency (RF) power to an electrical instrument, the generator comprising an RF output stage for the delivery of RF power to the instrument, a power supply for supplying power to the output stage and control circuitry sensing means for deriving a signal representative of the peak output voltage of the output stage and comparing the peak output voltage to a first voltage threshold and altering the output voltage if the peak output voltage exceeds the first voltage threshould.
